# EUROPÄISCHE PATENTANMELDUNG

(11) **EP 0 891 780 A2**
(43) Veröffentlichungstag der Anmeldung: **20.01.1999**
(21) Anmeldenummer: 98112879.6
(22) Anmeldetag: 10.07.1998
(51) Int. Cl.: A61K 47/48

(54) **Transfektionssystem, seine Herstellung und Verwendung in der somatischen Gentherapie**

(30) Priorität: 11.07.1997 DE 19729769
(71) Anmelder: Cardiogene Gentherapeutische Systeme AG, 40699 Erkrath (DE); Schrader, Jürgen, Prof. Dr., 40597 Düsseldorf (DE)
(72) Erfinder: Schrader, Jürgen, Prof. Dr., 40597 Düsseldorf (DE)
(74) Vertreter: Bösl, Raphael, Dr. rer. nat., Dipl.-Chem.

(57) **Zusammenfassung**

Die vorliegende Erfindung betrifft ein Transfektionssystem enthaltend einen oder mehrere Infiltrator-Katheter, eine oder mehrere Nukleinsäuren, und gegebenenfalls geeignete Hilfs- und/oder Zusatzstoffe, sowie seine Herstellung und Verwendung in der somatischen Gentherapie.

## Beschreibung

Die vorliegende Erfindung betrifft ein Transfektionssystem enthaltend einen oder mehrere Infiltrator-Katheter, eine oder mehrere Nukleinsäuren, und gegebenenfalls geeignete Hilfs- und/oder Zusatzstoffe, sowie seine Herstellung und Verwendung in der somatischen Gentherapie.

Die lokale gentherapeutische Behandlung vaskulärer Erkrankungen stellt in der interventionellen Kardiologie eine vielversprechende Perspektive dar, die beispielsweise den Wiederverschluß von Gefäßen (Restenose) nach einer mechanischen Aufdehnung des verschlossenen Gefäßes mit einem Ballonkatheter (sogenannte perkutane transluminale Koronarangioplastie; PTCA) verhindern könnte, da nach einer PTCA-Behandlung es heute immer noch in 30-40 % aller Fälle zu einer Restenose kommt.

Die systemische Gabe von Pharmaka hat trotz vielversprechender theoretischer Konzepte keine Verbesserung des Langzeiterfolges nach PTCA erbracht, vermutlich weil die aktuelle Konzentration des Therapeutikums im Bereich der behandelten Stenose nicht ausreichend war. Diese Ergebnisse haben zu der Entwicklung verschiedener Spezialkatheter geführt, die die lokale Behandlung eines geschädigten Gefäßabschnittes mit speziellen Pharmaka erlauben.

Ein Nachteil bei der lokalen Applikation von Medikamenten, insbesondere von Medikamenten mit geringem Molekulargewicht, mit Spezialkathetern ist jedoch ein schnelles Herausdiffundieren aus dem behandelten Gefäßabschnitt. Die gewünschte Depotbildung hält nur kurzfristig an.

Eine Verlängerung der therapeutischen Wirkung kann nun beispielsweise dadurch erreicht werden, daß therapeutisch wirksame Gene über einen somatischen Gentransfer lokal in die Gefäßwand übertragen und dort exprimiert werden.

Für den Gentransfer in die Gefäßwand sind im wesentlichen drei Komponenten notwendig:
- Ein therapeutisch wirksames Gen, das bei lokaler Expression beispielsweise zur Synthese eines Faktors führt, der die Restenosebildung hemmt.
- Ein Transfektionssystem, das
   (a) eine möglichst effiziente Transfektion der Gefäßwand mit dem therapeutischen Gen und
   (b) eine lokale Begrenzung der Transfektion ermöglicht.
- Eine effiziente Kathetertechnologie, die es erlaubt,
   (a) unter Einsatz: minimal invasiver Techniken gezielt einzelne Gefäßabschnitte auf hypo- bzw. Atraumatische Weise in vivo zu transfizieren,
   (b) in Kombination mit dem Transfektionssystem eine minimale Verteilung des therapeutischen Gens in den perivaskulären Baum bzw. in den Blutkreislauf zu gewährleisten, und
   (c) kurze Gefäßversclußzeiten zu garantieren.

Schrader, J. & Gödecke, A. (DE 44 11 402) fanden nun, daß die Transfektion mit dem Stickstoffmonoxid-Synthasegen in Form eines Liposomenkomplexes in Blutgefäße zu einer therapeutisch relevanten Hemmung der Gefäß-Stenose und der Restenose nach PTCA führt. Für die Transfektion mit Hilfe der DNA-Liposomenkomplexe wurde jedoch ein Polyethylen-Katheter verwendet, der den Blutstrom des Gefäßes für 15-20 Minuten unterbrach. Zudem führte die Transfektion in einigen Fällen zu einer Thrombose nach Wiedereröffnung des Gefäßes.

In der Literatur sind bereits einige andere Spezialkatheter beschrieben worden, mit denen das Problem einer lokalen Applikation von Pharmaka gelöst werden soll (Wilenksy, R.L. et al. (1993) Trends Cardiovasc. Med. 3(5), 163-170).

Beispielsweise erlaubt es der Doppelballonkatheter, einen definierten Gefäßabschnitt durch Inflation eines distal und eines proximal von dem zu transfizierenden Gefäßabschnitt gelegenen Ballons von der Zirkulation abzutrennen (siehe z. B. Nathan, A. & Edelman E. R. (1995) in Edelman, E. R. (ed.), "Frontiers in Cardiology; Molecular Interventions and Local Drug Delivery" Saunders Company Ltd., London, GB, 29-52). Das so isolierte Lumen wird dann mit dem Therapeutikum gefüllt, das durch Diffusion in die Gefäßwand gelangt. Nachteilig für diesen Katheter sind jedoch die langen Verschlußzeiten der Gefäße bei der Anwendung und man erreicht im wesentlichen nur die innersten Zellagen der Gefäße (Flugelman, M.Y. (1995), Thrombosis and Haemostasis, 74(1), 406-410). Daher ist dieser Kathetertyp für den somatischen Gentransfer nicht geeignet.

Der poröse Ballon wurde entwickelt, um ein Therapeutikum unter Druck durch Poren in einem inflatierten Ballon in die Gefäßwand zu pressen und so eine transmurale Verteilung des Therapeutikums zu erreichen. Die erforderlichen Hohen Drücke zur Injektion (2-5 atm.) führten aber häufig zu starken mechanischen Zerstörungen der Gefäßwand, die sich entweder in Dissektionen oder in der Entwicklung nekrotischer Zonen in der Media äußerten. Zudem ist die Transfektionseffizienz außerordentlich gering und nicht lokal begrenzt (Flugelman, M.Y. (1995), supra; Flugelman, M.Y. et al. (1992) Circulation, 85(3), 1110-1117). Daher schied dieser Kathetertyp ebenso für den somatischen Gentransfer aus.

Das Funktionsprinzip des Dispatch-Katheters ähnelt im Prinzip dem oben beschriebenen Doppelballonkatheter (siehe z. B. McKay, R. G. et al. (1994), Catheterization and Cardiovascular Diagnosis, 33, 181-188). Der zu behandelnde Gefäßabschnitt wird hier allerdings nicht durch zwei periphere Ballons vom Rest des Kreislaufs abgetrennt, sondern durch eine Spirale, die sich nach Inflation an die Gefäßwand anlegt. Durch Öffnungen im Katheterschaft zwischen den Spiralelementen werden die Vektoren in die abgeschlossene Kammer injiziert. Die Transfektion erfolgt durch Diffusion. Um die notwendige lange Transfektionszeit von ca. 30 Minuten zu ermöglichen ohne die distal gelegenen Bereiche von der Durchblutung abzuschneiden, wurde eine im Schaft des aufgeblasenen Katheters verlaufende Leitung gelegt, die den Blutfluß ermöglicht.

Der Nadelinjektionskatheter (NIC) ist gekennzeichnet durch drei Injektionsnadeln, die aus der abgerundeten Spitze eines Katheters ausgefahren werden können und dann in die Gefäßwand eindringen (siehe z. B. Gonschior, P. et al. (1995) Coronary Artery Disease, 6, 329-344). Über diese Nadeln konnten bereits Pharmaka in die Gefäßwand injiziert werden.

Die Handhabung des NIC ist jedoch schwierig, da keine einfache Kontrolle darüber möglich ist, wie weit die Nadeln aus dem Katheterkopf bereits ausgetreten sind, wodurch aber die Injektionstiefe definiert wird. Dies beinhaltet das Risiko einer Perforation der Gefäßwand und damit die Transfektion perivaskulärer Gewebe, aber auch einer Blutung aus dem Gefäß. Diese Gefahr ist besonders bei der Transfektion exzentrischer Plaques gegeben.

Janssens, S. et al., The Second Annual International Symposium, October 13-15, 1996, Cambridge, Massachusetts beschreiben einen Gentransfer mit Hilfe eines adenoviralen Vektors und eines nicht näher beschriebenen Infiltrator-Katheters. Nachteil dieses Transfektionssystems ist jedoch, daß bei adenoviralen Vektoren cytotoxische Effekte beobachtet wurden (Flugelman, M.Y. (1995), supra).

Aufgabe der vorliegenden Erfindung war es daher, ein Transfektionssystem zu finden, das den somatischen Gentransfer in Gefäße effektiv und auf möglichst schonende Art und Weise bewerkstelligen kann.

Ein Gegenstand der vorliegenden Erfindung ist daher ein Transfektionssystem enthaltend einen oder mehrere Infiltrator-Katheter, eine oder mehrere Nukleinsäuren in nicht-viraler Form, und gegebenenfalls geeignete Hilfs- und/oder Zusatzstoffe.

Der Infiltrator-Katheter ist ein Spezialkatheter, der für die intravaskuläre Injektion von Pharmaka in die Gefäßwand entwickelt wurde. Es handelt sich hierbei um einen Ballonkatheter, aus dessen Oberfläche Injektionsports (röhrenförmige, stollenartige Erweiterungen zur Verabreichung eines oder mehrerer Wirkstoffe) ragen. Die Höhe der Injektionsports beträgt üblicherweise ca. 100-500 µm, vorzugsweise ca. 100-250 µm, insbesondere ca. 100 µm und die Anzahl der Injektionsports je Ballon beträgt üblicherweise ca. 5 x 7, vorzugsweise ca. 3 x 7. Durch Inflatieren des Ballons bei üblicherweise ca. 2 atm werden diese Injektionsports in die Gefäßwand gedrückt.

Über die Injektionsports können dann im allgemeinen bis zu ca. 500 µl, vorzugsweise ca. 250-300 µl Wirkstoff bzw. Wirkstoffe unter niedrigem Druck, vorzugsweise ca. 100-200 mm Hg, insbesondere ca. 150 mm Hg, in die Gefäßwand injiziert werden.

Üblicherweise handelt es sich bei dem Katheter um einen mehrlumigen, insbesondere um einen doppellumigen, besonders bevorzugt um einen dreilumigen Katheter.

Der doppellumige Katheter besteht im allgemeinen aus einem röhrenförmigen Schaft und dem genannten aufblasbaren Ballon, der an einer geeigneten Stelle den Schaft umschließt. Der Schaft enthält an der Stelle des Ballons eine oder mehrere Öffnungen, über die der Ballon aufgeblasen und der Wirkstoff in den Ballon gelangen kann. Von dort gelangt der Wirkstoff über die Injektionsports in die Gefäßwand. Besonders bevorzugt ist ein zweilumiger Infiltrator-Katheter wie in U.S. Patent 5,112,305 oder U.S. Patent 5,242,397 beschrieben.

Besonders bevorzugt ist ein dreilumiger Infiltrator-Katheter, der einen zentralen röhrenförmigen Schaft, einen aufblasbaren Ballon, der den Schaft an einer geeigneten Stelle umschließt, und eine röhrenförmige Manschette enthält, die an der Stelle des aufblasbaren Ballons die genannten Injektionsports enthält. Nach der Positionierung des Katheters wird der Ballon inflatiert, wobei die genannte Manschette mit den Injektionsports gegen die Gefäßwand gedrückt wird. Anschließend wird der Wirkstoff in die röhrenförmige Manschette verbracht, um von dort über die Injektionsports in die Gefäßwand zu gelangen. Besonders bevorzugt als ein dreilumiger Infiltrator-Katheter ist ein Katheter wie in EP 0 753 322 A1 oder EP 0 768 098 A2 beschrieben.

Im allgemeinen enthält das erfindungsgemäße Transfektionssystem zusätzlich einen Führungsdraht, der eine korrekte Positionierung des Katheters ermöglicht.

Die Nukleinsäure des erfindungsgemäßen Transfektionssystems liegt in nichtviraler Form, vorzugsweise als einzel- oder doppelsträngige DNA oder als RNA, beispielsweise als nackte Nukleinsäure oder zusammen mit weiteren nicht-viralen Komponenten, vor.

Der Begriff "nicht-viral" bedeutet gemäß der vorliegenden Erfindung, daß die Nukleinsäure nicht mit Hilfe gentechnisch veränderter viraler Vektoren, wie z.B. retrovirale, adenovirale oder Adeno-assoziierte virale Vektoren, transfiziert wird. Die Verwendung von z.B. Sendai-Viren in Form von Virosomen (s.u.) wird durch den Begriff "nicht-viral" nicht ausgeschlossen.

Als nackte Nukleinsäure eignet sich beispielsweise eine Nukleinsäure in Form einer Plasmid-DNA oder ein sogenanntes Antisense-Oligonukleotid (siehe z. B. Uhlmann, E. & Peyman, A. (1990) Chemical Reviews, 90, 543-584, No. 4).

Gentherapeutisch wirksame Nukleinsäuren lassen sich auch dadurch erhalten, daß man die gewünschte Nukleinsäure mit weiteren nicht-viralen Komponenten, vorzugsweise Liposomen komplexiert, da damit eine sehr hohe Transfektionseffizienz, insbesondere der Gefäßwand, erreicht werden kann (siehe z. B. DE 44 11 402 A1). Besonders vorteilhaft ist die Transfektion mit Nukleinsäure-Liposomen-Komplexen mit Hilfe von Sendai-Viren in Form von sogenannten HVJ-Liposomen (Virosomen), da hierdurch die Transfektionsrate noch weiter gesteigert werden kann.

Bei der Lipofektion werden kleine unilamellare Vesikel aus beispielsweise kationischen Lipiden durch Ultraschallbehandlung der Liposomensuspension hergestellt. Die Nukleinsäure wird ionisch auf der Oberfläche der Liposomen gebunden, und zwar in einem solchen Verhältnis, daß eine positive Nettoladung verbleibt und die Nukleinsäure zu 100% von den Liposomen komplexiert wird. Neben den von Felgner et al. (Felgner, P. L. et al. (1987), Proc. Natl. Acad. Sci USA, 84, 7413 - 7414) eingesetzten Lipidmischungen DOTMA (1,2-Dioleyloxypropyl-3-trimethyl-ammoniumbromid) und DOPE (Dioleoylphosphatidylethanolamin) wurden inzwischen zahlreiche neue Lipidformulierungen synthetisiert und auf ihre Effizienz der Transfektion verschiedener Zellinien getestet (Behr, J.P. et al. (1989), Proc. Natl. Acad. Sci. USA, 86, 6982 - 6986; Felgner, J.H. et al. (1994) J. Biol. Chem., 269, 2550 - 2561; Gao, X. & Huang, L. (1991), Biochim. Biophys. Acta, 1189, 195 - 203). Beispiele der neuen Lipidformulierungen sind DOTAP N-[1-(2,3-Dioleoyloxy)propyl]-N,N,N-trimethylammoniumethylsulfat oder DOGS (TRANSFECTAM; Dioctadecylamidoglycylspermin). Ein Beispiel für die Herstellung von DNA-Liposomenkomplexen aus Phosphatidylcholin, Phosphatidylserin und Cholesterin und deren erfolgreiche Anwendung in der Transfektion von Gefäßwänden mit Hilfe von Sendai-Viren ist in der DE 44 11 402 beschrieben.

Besonders vorteilhaft ist es, wenn der Nukleinsäure-Liposomenkomplex Nukleinsäure-Bindeproteine, beispielsweise chromosomale Proteine, vorzugsweise HMG-Proteine (High Mobility Group Proteine), insbesondere HMG-1 oder HMG-2, oder nukleosomale Histone wie H2A, H2B, H3 oder H4 enthält, da hierdurch die Expression der gewünschten Nukleinsäure mindestens 3-10fach gesteigert werden kann. Die chromosomalen Proteine können beispielsweise aus Kalbsthymus oder Rattenleber nach allgemein bekannten Verfahren isoliert oder gentechnisch hergestellt werden. Humanes HMG-1 kann beispielsweise nach dem Fachmann bekannten Methoden besonders einfach gentechnisch unter Verwendung der humanen cDNA-Sequenz aus Wen, L. et al. (1989) Nucleic Acids Res., 17(3), 1197-1214, hergestellte werden.

Die gewünschte Nukleinsäure ist im allgemeinen eine Nukleinsäure, die für ein therapeutisch wirksames Genprodukt kodiert.

Beispiele von Nukleinsäuren, die für ein therapeutisch wirksames Genprodukt kodiert, sind das Stickstoffmonoxid-Synthase-Gen, insbesondere ein Gen, das für die induzierbare Stickstoffmonoxid-Synthase kodiert (siehe z. B. DE 44 11 402 A1), das Erythropoietin-Gen (siehe z. B. EP 0 148 605 B1), das Insulin-Gen (siehe z. B. EP 0 001 929 B1) oder die Gene kodierend für Blutgerinnungsfaktoren, Interferone, Cytokine, Hormone, Wachstumsfaktoren usw. Besonders bevorzugt sind Gene, die für im Blut vorkommende Proteine kodieren. Die erfindungsgemäße somatische Gentherapie der Gefäßwand kann auf besonders einfache und dauerhafte Weise beispielsweise eine krankhafte Mangelerscheinung, wie z. B. einen Mangel an Insulin bei Diabetiker, einen Mangel am Faktor VIII bei Bluter, einen Mangel an Erythropoietin bei Nierenpatienten, einen Mangel an Thrombopoietin oder einen Mangel an Somatostatin bei Zwergwuchs, durch eine Erhöhung der Plasmakonzentrationen des jeweiligen Wirkstoffes beheben bzw. lindern. Die vorliegende Erfindung ist daher nicht nur auf die Therapie rein vaskulärer Erkrankungen, wie z. B. die Arteriosklerose, Stenose oder Restenose, beschränkt, sondern ist allgemein anwendbar.

Für die beschriebene gentherapeutische Anwendung ist es auch von Vorteil, wenn der Teil der Nukleinsäure, der für das Polypeptid kodiert, ein oder mehrere nicht-kodierende Sequenzen einschließlich Intronsequenzen, vorzugsweise zwischen Promotor und dem Startcodon des Polypeptids, und/oder eine poly A-Sequenz, insbesondere die natürlich vorkommende poly A-Sequenz oder eine SV40 Virus polyA-Sequenz, vor allem am 3'-Ende des Gens enthält, da hierdurch eine Stabilisierung der mRNA in der Zelle erreicht werden kann (Jackson, R. J. (1993) Cell, 74, 9-14 und Palmiter, R. D. et al. (1991) Proc. Natl. Acad. Sci. USA, 88, 478-482).

Das erfindungsgemäße Transfektionssystem laßt sich auf einfache Weise dadurch herstellen, daß ein oder mehrere der oben beschriebenen oder käuflich zu erwerbenden Infiltrator-Katheter, ein oder mehrere der oben beschriebenen Nukleinsäuren, gegebenenfalls geeignete Hilfs- und/oder Zusatzstoffe und gegebenenfalls ein Führungsdraht zusammengestellt werden.

Als Hilfs- und/oder Zusatzstoffe eignen sich beispielsweise dem Fachmann bekannte Stabilisatoren, wie Nukleaseinhibitoren, vorzugsweise Komplexbildner wie EDTA, Proteaseinhibitoren oder Erdalkalilösungen insbesondere bei Verwendung von Sendai-Viren im erfindungsgemäßen Transfektionssystem, wie z. B. eine CaCl₂-Lösung, die vorzugsweise vor einer Transfektion mit Virosomen hinzugegeben wird.

Der beschriebene Infiltrator-Katheter eignet sich in Form des erfindungsgemäßen Transfektionssystems in vorteilhafter Weise für die somatische Gentherapie, insbesondere zur Behandlung von Gefäßerkrankungen, genetisch bedingten Erkrankungen und/oder durch Gentransfer therapierbare Erkrankungen einschließlich deren Prävention, wie beispielshaft oben bereits näher beschrieben.

Daher erstreckt sich die vorliegende Erfindung auch auf die Verwendung eines Infiltrator-Katheters zur Herstellung eines erfindungsgemäßen Transfektionssystems vorzugsweise für die somatische Gentherapie. Die vorliegende Erfindung erstreckt sich ferner auf die Verwendung des erfindungsgemäßen Transfektionssystems für den somatische Gentransfer, insbesondere zur Behandlung oder Prävention der beschriebenen Erkrankungen.

Der unerwartete Vorteil der vorliegenden Erfindung liegt darin, daß im Gegensatz zu anderen in der Literatur beschriebenen Transfektionssystemen, die für die lokale Applikation von Arzneimitteln entwickelt worden sind, ein Infiltrator-Katheter für die Übertragung von einer oder mehreren Nukleinsäuren in der somatischen Gentherapie besonders gut gemäß der vorliegenden Erfindung geeignet ist. Der Infiltrator-Katheter konnte im Gefäß genau positioniert werden, führte bei Inflation des Ballons zu einem dichten Verschluß des Gefäßes, so daß die Injektionsports durch den Ballon in die Gefäßwand gedrückt wurden und die Injektion der Nukleinsäure(n) unter geringem Druck wirkungsvoll erfolgen konnte, was nur zu minimalen Veränderungen der Gefäßwand führte. Hierdurch konnte bei kurzen Verschlußzeiten des Gefäßes (maximal ca. 30 Sekunden) eine effiziente Transfektion der Gefäßwand und eine hohe und dauerhafte Expression der gewünschten Nukleinsäure erreicht werden.

Die folgenden Figuren und Beispiele sollen die Erfindung näher erläutern ohne sie darauf zu beschränken.

### BESCHREIBUNG DER FIGUREN

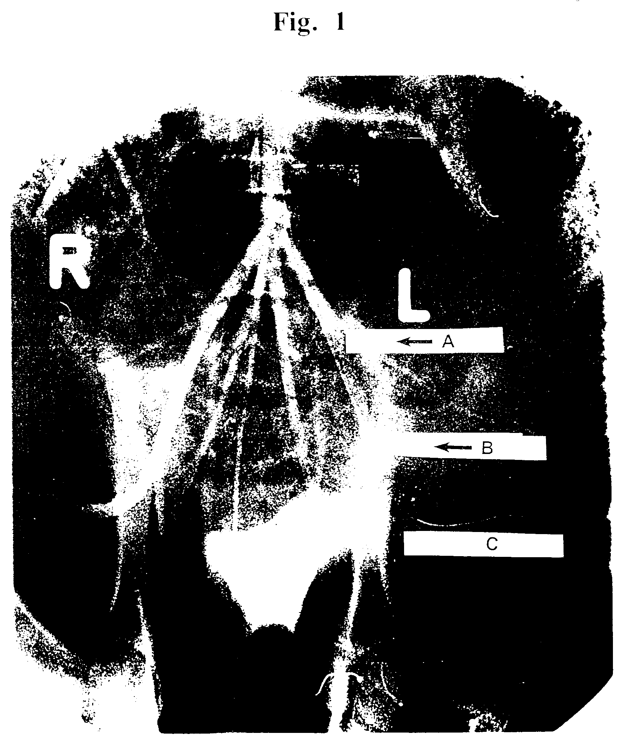

Fig. 1 zeigt eine Röntgenaufnahme der Beckenstrombahn des Schweins während der Transfektion der linken A. iliaca. Die Aufnahme zeigt den inflatierten Infiltrator-Katheter (B) in der A. iliaca (A) des Schweins. Distal vom Katheter ist der Führungsdraht zu erkennen, der zur Einführung des Katheters benutzt wurde (C). Der dichte Verschluß des Gefäßes durch den inflatierten Ballon ist an der fehlenden Kontrastierung der distal gelegenen Femoralarterien zu erkennen (vgl. rechte Seite).

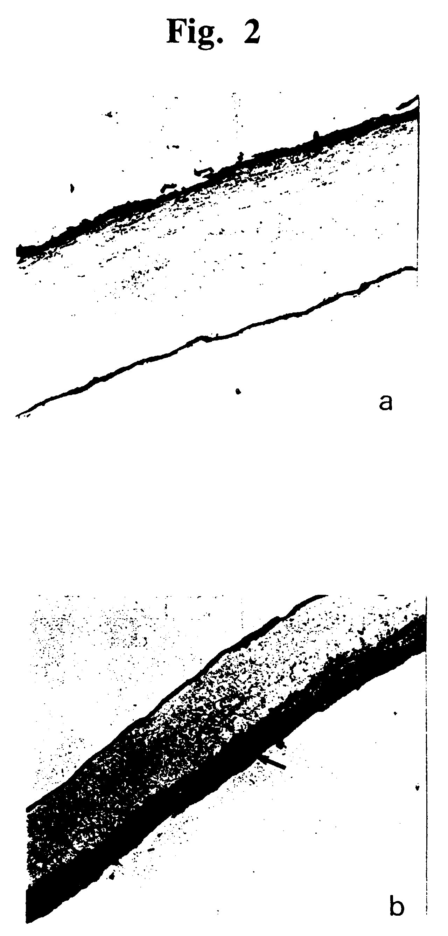

Fig. 2 a und b zeigen den immunhistochemischen Nachweis der iNOS Expression in Iliacalarterien des Schweins nach Transfektion mit dem Kontrollvektor (Fig. 2 a) bzw. mit dem pSCMV-iNOS-Vektor (Fig. 2 b). Die dunkle Färbung in Fig. 2 b (Pfeil) weist auf eine starke iNOS Expression hin.

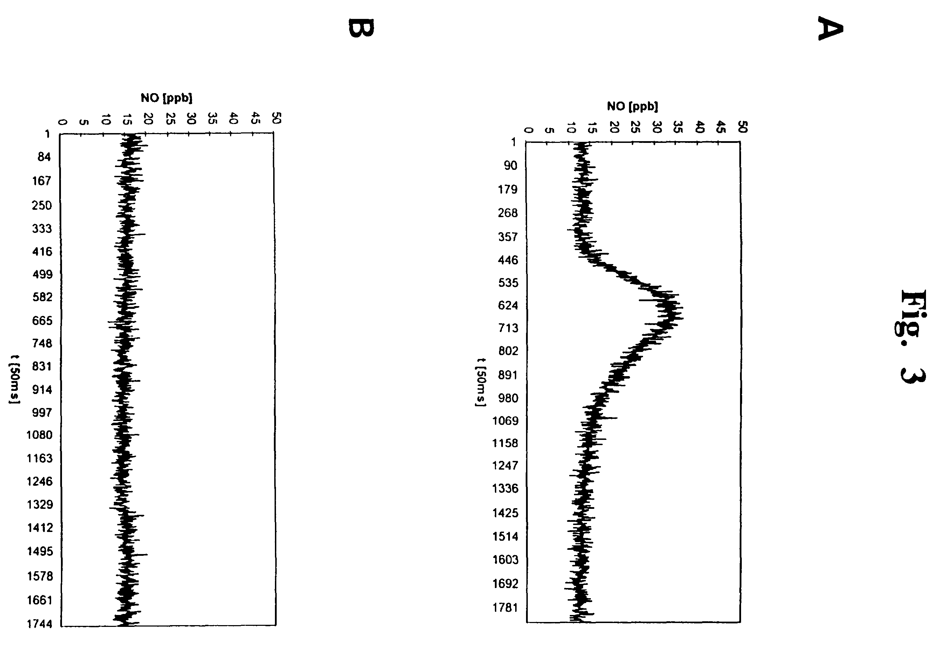

Fig. 3 A und B zeigen den Nachweis der NOx-Akkumulation im Kulturüberstand von Gefäßen, die entweder mit dem pSCMV-iNOS-Vektor (A) bzw. mit dem Kontrollvektor (B) transfiziert wurden. Während im Kontrollsegment (Fig. 3 B) kein NOx-Signal feststellbar ist, findet man im transfizierten Gefäß eine deutliche NOx-Freisetzung (Fig. 3 A). (ppB = parts per billion)

### BEISPIEL

### Expression des induzierbaren Stickstoffmonoxid-Synthase-Gens in der Aorta femoralis des Schweins

### 1. Transfektionsprotokoll

### 1.1 Vorbereitung der DNA

200 µg des Gentransfervektors pSCMV-iNOS, der für die induzierbare Stickstoffmonoxid-Synthase der Maus kodiert (DE 44 11 402 A1), in einer Konzentration von 2µg/µl gelöst in TE-Puffer (10 mM Tris-HCl, pH 7.4) wurden mit 64 µg HMG-1 Protein aus Kalbsthymus gelöst in BSS-Puffer (140 mM NaCl, 5,4 mM KCl, 10 mM Tris·Cl,pH 7,6) gemischt und die Lösung mit BSS-Puffer auf ein Endvolumen von 200 µl eingestellt. Die erhaltene Lösung wurde für 30 min bei 37 °C inkubiert.

### 1.2 Liposomenherstellung

Je 5 mg eines Lipidgemisches aus Phosphatidylcholin (PC), Cholesterin (C) und Phosphatidylserin (PS) mit einem Gewichtsverhältnis in der Mischung von 4,8:2:1 wurden in 2 ml Diethylether gelöst. Zu den gelösten Lipiden wurden 200 µl der in Beispiel 1.1 hergestellten DNA-Lösung gegeben. Das Gemisch wurde dann 2 min durch Vortexen homogenisiert und anschließend für 10 sec. in einem Ultraschallbad beschallt. Anschließend wurde der Ether bei 37 °C in einem Rotationsverdampfer evaporiert. Die verbleibende Emulsion wurde daraufhin ca. 2 min bis zum Auftreten einer Opaleszenz gevortext.

### 1.3 Vorbereitung der Sendai-Viren

Sendai-Viren wurden nach Standardverfahren in der Chorioallantoisflüssigkeit befruchteter Hühnereier angezüchtet (Nakanishi, M. et al. (1985) Exp. Cell. Res., 159(2), 399 - 409). Die Viren wurden anschließend durch folgendes Zentrifugationsverfahren in einem Sorvall GSA-Rotor bei 4 °C gereinigt:
- 1. Chorioallantoisflüssigkeit:: 10 min bei 3.000 rpm
- 2. Überstand:: 30 min bei 13.000 rpm
- 3. Pellet:: in BSS-Puffer resuspendiert
- 4. Suspension:: 10 min bei 3.000 rpm
- 5. Überstand:: 30 min bei 13.000 rpm
- 6. Pellet:: in BSS-Puffer resuspendiert.

Anschließend wurde der Titer auf 16.000 Hämagglutinations-Einheiten (HAU)/ml eingestellt. Kurz vor der Verwendung wurden die Viren durch UV-Bestrahlung bei 11J/m²·s inaktiviert.

### 1.4 Herstellung und Reinigung der Virosomen (HVJ-Liposomen)

1 ml der gemaß Beispiel 1.3 hergestellten und UV-inaktivierten Viren (16.000 HAU) wurden mit den gemäß Beispiel 1.2 hergestellten Liposomen gemischt und 10 min auf Eis inkubiert. Anschließend wurde die Suspension zur Fusion der Viren mit den Liposomen 60 min lang auf einem Rundschüttler (120 rpm, 37 °C) geschüttelt. Die gebildeten Virosomen wurden danach von den nicht-eingebauten Viren durch Ultrazentrifugation über ein Saccharosekissen (30% Saccharose) bei 28.000 rpm in einem TH641-Rotor, Sorvall Instruments, bei 4 °C abgetrennt. Die Virosomen bandierten dabei auf dem Saccharosekissen und die nicht-eingebauten Viren sedimentierten auf den Boden des Röhrchens. Die Virosomen wurden anschließend abgenommen und bis zur Transfektion auf Eis gelagert. Kurz vor der Durchführung der Transfektion wurde CaCl₂ zu einer Endkonzentration von 2 mM hinzugegeben.

### 1.5 Transfektion

Die Transfektion erfolgte durch Injektion von 300 µl der gemäß Beispiel 1.4 hergestellten Virosomen (8-10 µg pSCMV-iNOS eingeschlossen in HVJ-Liposomen, ca. 1.000 HAU) in die A. iliaca bei Münchner Minischweinen über die beschriebenen Katheter. Die Injektionsdauer betrug etwa 10 sec. Als Kontrolle wurden Virosomen eingesetzt, die den vektor pSCMV2 (DE 44 11 402 A1) enthielten.

### 2. Operative Eingriffe

Die linke A. carotis wurde mittels eines chirurgischen Eingriffs freigelegt und nach modifizierter Seldinger Technik eine 7-F bzw. 9-F Schleuse eingeführt. Über diese Schleuse erfolgten alle weiteren angiographischen oder interventionellen Schritte. Zunächst wird über einen Kalibrierungs-Pig-tail Katheter eine Übersichtsangiographie der distalen Aorta und der Beckengefäße angefertigt. Anschließend wurde der Katheter zur lokalen Transfektion über einen Führungsdraht bis in die Femoralarterien vorgeschoben. Die Transfektionslösung wurde dann über einen seitenständigen Injektionsport entsprechend der Angaben des Herstellers für die lokale Applikation von Pharmaka in das Gefäß eingebracht. Dabei wurden in demselben Tier Expressionsvektor und Kontrollvektor in die rechte bzw. linke A. femoralis injiziert. Nach Entfernen des Katheters wurde die A. carotis durch eine Gefäßnaht verschlossen. Anschließend erfolgte der Wundverschluß.

### 3. Immunhistochemischer Nachweis

Gefrierschnitte (8 µm) der transfizierten Gefäßwandabschnitte wurden mit monoklonalen anti-iNOS-Antikörpern (Transduction Laboratories, #N32020) umgesetzt. Der Nachweis der Antikörperbindung erfolgte über einen Streptavidin/Peroxidase Komplex anhand von biotinylierten anti-Maus-Antikörpern (Vector Laboratories, #BA-2000) mit dem Vecta Stain Elite Kit (Vector Laboratories #PK-6100; ).

### 4. Biochemischer Nachweis

Zusätzlich zur Immunhistochemie wurde direkt eine erhöhte NO-Bildung durch die transfizierten Gefäße anhand der Nitrat/Nitrit-(NOx )Akkumulation im Kulturüberstand explantierter transfizierter Gefäßabschnitte nachgewiesen. Transfizierte Gefäßabschnitte wurden in Krebs-Henseleitpuffer inkubiert (in mmol/L: 116 NaCl; 4,6 KCl; 1,1 MgSO₄; 24,9 NaHCO₃; 2,5 CaCl₂; 1,2 KH₂PO₄; 10 Glucose und 0,5 EDTA äquilibriert mit 95% O₂ und 5% CO₂ (pH 7,4; 37 °C)). Die Inkubation erfolgte über 24 h bei 37 °C. Die NOx Akkumulation wurde nach Reduktion von Nitrat + Nitrit zu NO mit Hilfe des Chemilumineszens-Verfahrens bestimmt (Nitric Oxide Analyzer NOA 280 Sievers Inc., USA).

### 5. Ergebnisse

### 5.1 Der Dispatch-Katheter

Bei der Verwendung eines Dispatch-Katheters gelang keine effiziente Transfektion, da eine dichtschließende isolierte Kammer durch Inflatieren der Spiralelemente nicht hergestellt werden konnte. Die Injektion der Vektoren über den Katheterschaft führte zu keinerlei Drucksteigerung, die bei einer geschlossenen Kammer zu erwarten gewesen wäre. Die Undichtigkeit der Kammer wurde durch das schnelle Abfließen von Röntgenkontrastmittel aus der Kammer nachgewiesen. Der Dispatch-Katheter ist daher für einen lokalen Gentransfer nicht geeignet.

### 5.2 Der Infiltrator-Katheter

Im Gegensatz zum Dispatch-Katheter oder Nadelinjektionskatheter erlaubte der Infiltrator-Katheter (Barath Drug Delivery Catheter mit 3 x 7 Injektionsports in der Längsachse mit einer zylinderartigen Form der Ports, die nach oben leicht konisch zuläuft, Model No. DD140015, Interventional Technologies Europe Ltd., Irland) eine genaue Positionierung (siehe Fig. 1) und führte bei Inflation des Ballons zu einem dichten Verschluß des Gefäßes.

Wie Fig. 2 zeigt, konnte in den transfizierten Gefäßabschnitten eine deutliche Immunreaktivität nachgewiesen werden, die bis zu 50 % des Gefäßwanddurchmessers erreichte. Eine entsprechende Färbung fehlte in den Kontroll-transfizierten Gefäßen, so daß dieser Nachweis spezifisch die Expression der iNOS erfaßte.

Fig. 3 zeigt, daß nach Transfektion eine deutlich erhöhte NOx Freisetzung im Vergleich zu Abschnitten, die mit dem Kontrolvektor transfiziert wurden, erhalten wurde.

Mit dem Infiltrator-Katheter konnte somit eine effiziente in vivo Transfektion der Gefäßwand, die bis zu 50 % des Gefäßwanddurchmessers erreichte, bei kurzen Verschlußzeiten der Gefäße (max. 30 s) gezeigt werden.

## Patentansprüche

1. Transfektionssystem enthaltend
(a) einen oder mehrere Infiltrator-Katheter;
(b) eine oder mehrere Nukleinsäuren in nicht-viraler Form; und gegebenenfalls
(c) geeignete Hilfs- und/oder Zusatzstoffe.

2. Transfektionssystem nach Anspruch 1, dadurch gekennzeichnet, daß der Infiltrator-Katheter einen oder mehrere Injektionsports mit einer Höhe von ca. 100-500 µm enthält.

3. Transfektionssystem nach Anspruch 1 oder 2, dadurch gekennzeichnet, daß der Infiltrator-Katheter ca. 5 x 7 Injektionsports enthält.

4. Transfektionssystem nach einem der Ansprüche 1 bis 3, dadurch gekennzeichnet, daß der Infiltrator-Katheter ein mehrlumiger Katheter ist.

5. Transfektionssystem nach einem der Ansprüche 1 bis 4, dadurch gekennzeichnet, daß der Infiltrator-Katheter einen zentralen röhrenförmigen Schaft, einen aufblasbaren Ballon, der den Schaft an einer geeigneten Stelle umschließt, und eine röhrenförmige Manschette enthält, die an der Stelle des aufblasbaren Ballons einen oder mehrere Injektionsports enthält.

6. Transfektionssystem nach einem der Ansprüche 1 bis 5, dadurch gekennzeichnet, daß das Transfektionssystem zusätzlich einen Führungsdraht enthält.

7. Transfektionssystem nach einem der Ansprüche 1 bis 6, dadurch gekennzeichnet, daß die Nukleinsäure in nackter Form oder zusammen mit weiteren nicht-viralen Komponenten vorliegt.

8. Transfektionssystem nach einem der Ansprüche 1 bis 7, dadurch gekennzeichnet, daß die Nukleinsäure in Form einer einzel- oder doppelsträngigen DNA oder RNA, vorzugsweise in Form einer Plasmid-DNA oder eines Antisense-Oligonukleotids vorliegt.

9. Transfektionssystem nach einem der Ansprüche 1 bis 8, dadurch gekennzeichnet, daß die Nukleinsäure in Form eines Nukleinsäure-Liposomen-Komplexes vorliegt.

10. Transfektionssystem nach Anspruch 9, dadurch gekennzeichnet, daß der Nukleinsäure-Liposomen-Komplex auch ein oder mehrere Nukleinsäure-Bindeproteine enthält.

11. Transfektionssystem nach einem der Ansprüche 1 bis 10, dadurch gekennzeichnet, daß die Nukleinsäure für ein therapeutisch wirksames Genprodukt kodiert.

12. Transfektionssystem nach Anspruch 11, dadurch gekennzeichnet, daß das therapeutisch wirksame Genprodukt eine Stickstoffmonoxid-Synthase ist.

13. Transfektionssystem nach Anspruch 11 oder 12, dadurch gekennzeichnet, daß die für ein therapeutisch wirksames Genprodukt kodierende Nukleinsäure eine oder mehrere nicht-kodierende Sequenzen und/oder eine polyA-Sequenz enthält.

14. Verfahren zur Herstellung eines Transfektionssystems gemäß einem der Ansprüche 1 bis 13, dadurch gekennzeichnet, daß ein oder mehrere Katheter gemäß einem der Ansprüche 1 bis 5, eine oder mehrere Nukleinsäuren in nicht-viraler Form und gegebenenfalls geeignete Hilfs- und/oder Zusatzstoffe und gegebenenfalls ein Führungsdraht zusammengestellt werden.

15. Verwendung eines Transfektionssystems gemäß einem der Ansprüche 1 bis 13 zur Herstellung eines Arzneimittels für die somatische Gentherapie.

16. Verwendung nach Anspruch 15 zur Behandlung von Gefäßerkrankungen, genetisch bedingten Erkrankungen und/oder durch Gentransfer therapierbare Erkrankungen einschließlich deren Prävention.

17. Verwendung eines Katheters gemäß einem der Ansprüche 1 bis 5 zur Herstellung eines Transfektionssystems gemäß einem der Ansprüche 1 bis 13.
